(19) **Europäisches Patentamt European Patent Office Office européen des brevets**

(11) **EP 2 406 400 B1**

(12) **EUROPEAN PATENT SPECIFICATION**

(45) Date of publication and mention of the grant of the patent:
**13.07.2016 Bulletin 2016/28**

(51) Int Cl.:
**C12Q 1/68** (2006.01)   **G06F 19/00** (2011.01)
**G06F 19/20** (2011.01)

(21) Application number: **10751309.5**

(22) Date of filing: **09.03.2010**

(86) International application number:
**PCT/US2010/026726**

(87) International publication number:
**WO 2010/104893 (16.09.2010 Gazette 2010/37)**

(54) **METHODS FOR THE DETERMINATION OF A COPY NUMBER OF A GENOMIC SEQUENCE IN A BIOLOGICAL SAMPLE**

VERFAHREN ZUR BESTIMMUNG DER KOPIEZAHL EINER GENOMSEQUENZ IN EINER BIOLOGISCHEN PROBE

MÉTHODES DE DÉTERMINATION DU NOMBRE DE COPIES D'UNE SÉQUENCE DE GÉNOME DANS UN ÉCHANTILLON BIOLOGIQUE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **09.03.2009 US 158718 P**

(43) Date of publication of application:
**18.01.2012 Bulletin 2012/03**

(73) Proprietor: **Life Technologies Corporation Carlsbad, CA 92008 (US)**

(72) Inventors:
  • **LEONG, Harrison M.**
   **San Francisco**
   **California 94118 (US)**
  • **BARBACIORU, Catalin**
   **Fremont**
   **California 94536 (US)**
  • **JANAWAY, Gordon A.**
   **Hayward**
   **California 94542 (US)**

(74) Representative: **Weber, Birgit Life Technologies GmbH Frankfurter Strasse 129 B 64293 Darmstadt (DE)**

(56) References cited:
   **WO-A2-2007/036258   US-A1- 2005 064 476**
   **US-A1- 2007 259 351   US-B1- 6 180 349**

• **THOMAS D SCHMITTGEN ET AL: "Analyzing real-time PCR data by the comparative CT method", NATURE PROTOCOLS, vol. 3, no. 6, 1 June 2008 (2008-06-01), pages 1101-1108, XP055137608, ISSN: 1754-2189, DOI: 10.1038/nprot.2008.73**
• **RONALD T KURNIK ED - CELLER B G ET AL: "Rotation Algorithm for Determining Cycle Thresholds in Real-Time Polymerase Chain Reactions", 2007 ANNUAL INTERNATIONAL CONFERENCE OF THE IEEE ENGINEERING IN MEDICINE AND BIOLOGY SOCIETY : [EMBC '07] ; LYON, FRANCE, 22 - 26 AUGUST 2007 ; [IN CONJUNCTION WITH THE BIENNIAL CONFERENCE OF THE SOCIÉTÉ FRANÇAISE DE GÉNIE BIOLOGIQUE ET MÉDICAL (SFGB, 1 August 2007 (2007-08-01), pages 1977-1980, XP031149863, ISBN: 978-1-4244-0787-3**
• **LIVAK K J ET AL: "Analysis of Relative Gene Expression Data Using Real-Time Quantitative PCR and the 2-deltadeltaCY Method", METHODS, ACADEMIC PRESS, vol. 25, 1 January 2001 (2001-01-01), pages 402-408, XP003015637, ISSN: 1046-2023, DOI: 10.1006/METH.2001.1262**
• **GINZINGER DG. ET AL.: 'Measurement of DNA copy number at microsatellite loci using quantitative PCR analysis' CANCER RES. vol. 60, no. 19, 01 October 2000, pages 5405 - 5409, XP002187743**

EP 2 406 400 B1

• **IWAO-KOIZUMI K. ET AL.: 'A novel technique for measuring variations in DNA copy-number: competitive genomic polymerase chain reaction' BMC GENOMICS vol. 8, no. 206, 02 July 2007, XP021028020**

## Description

### FIELD

The field of disclosure relates to methods for determining the copy number of a biological sample with a defined confidence.

### BACKGROUND

The polymerase chain reaction (PCR) represents an extensive family of chemistries that have produced numerous types of assays of impact in biological analysis. Accordingly, concomitant to the innovation of assays for this family of chemistries has been the innovation of computational methods matched to the objectives of the various PCR-based assays.

For example, one type of computational method suited to various types of quantitative PCR (qPCR) assays is often referred to as the comparative threshold cycle ($C_t$) method. As one of ordinary skill in the art is apprised, the cycle threshold, $C_t$, indicates the cycle number at which an amplified target genomic sequence; either a gene or genomic sequence of interest, reaches a fixed threshold. A relative concentration of a target genomic sequence; either a gene or genomic sequence of interest, may be determined using $C_t$, determinations for the target genomic sequence, a reference genomic sequence; of which for many qPCR assays may be either an endogenous or exogenous reference genomic sequence, and additionally, a calibrator sequence. After normalizing the $C_t$, data for the target gene sequence and the calibrator gene sequence to the reference gene sequence samples, under the assumption that the efficiencies of the reactions are equal and essentially 100%, one of ordinary skill in the art would recognize the calculation for the comparative $C_t$ method as:

$$X_{N,t} / X_{N,c} = 2^{-\Delta\Delta C_t};$$

where

$X_{N,t} / X_{N,c}$ = is the relative concentration of the target in comparison to the calibrator; and
$\Delta\Delta C_t$ = is the normalized difference in threshold cycles for the target and the calibrator

In practice, the efficiency of the PCR process may not be exactly 100%, as the concentration of genetic material may not double at every cycle. Factors that may affect the efficiency of an amplification reaction may include, for example, reaction conditions such as the difference in the detection limit for the dye used for a target genomic sequence versus the dye for the reference, or in inherent differences in the sequence context of the target genomic sequence and a reference genomic sequence. However, as assays are optimized to ensure the highest efficiencies, any deviations from the assumption of 100% efficiency are generally small. In addition to possible deviations from ideality, there are variations of replicate samples of the same sequence, due to variations contributions in an assay system from both the chemistry and instrumentation.

Accordingly, various methods for the determination of a gene copy use statistical models to assign a copy number to a sample in a population of samples, and determine a confidence value to the assignment. Such methods take into account various assay deviations and variations. Unlike the comparative $C_t$ method, or $\Delta\Delta C_t$ method, as it is often referred and which is reviewed in Schmittgen et al., Nature Protocols 3, 1101 - 1108 (2008), various methods for the determination of a gene copy number utilize the information in $\Delta C_t$ determinations of samples, and therefore do not require the use of a calibration sample data.

### BRIEF DESCRIPTION OF THE DRAWINGS

FIG. 1 is a flow chart that depicts various embodiments of methods for the determination of the copy number for a biological sample.

FIG. 2 depicts various embodiments of an apparatus useful in the generation of data for a biological sample for which a copy number determination is desired.

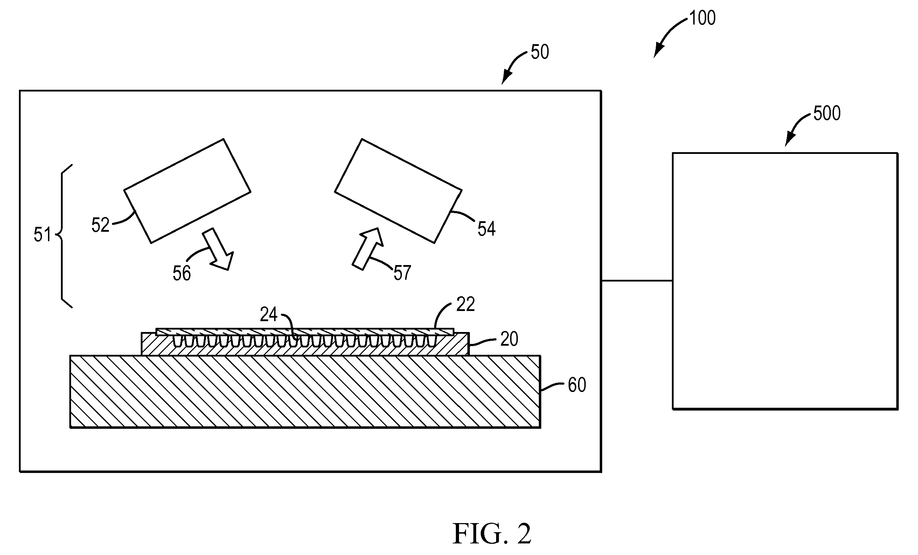

**FIG. 2** depicts

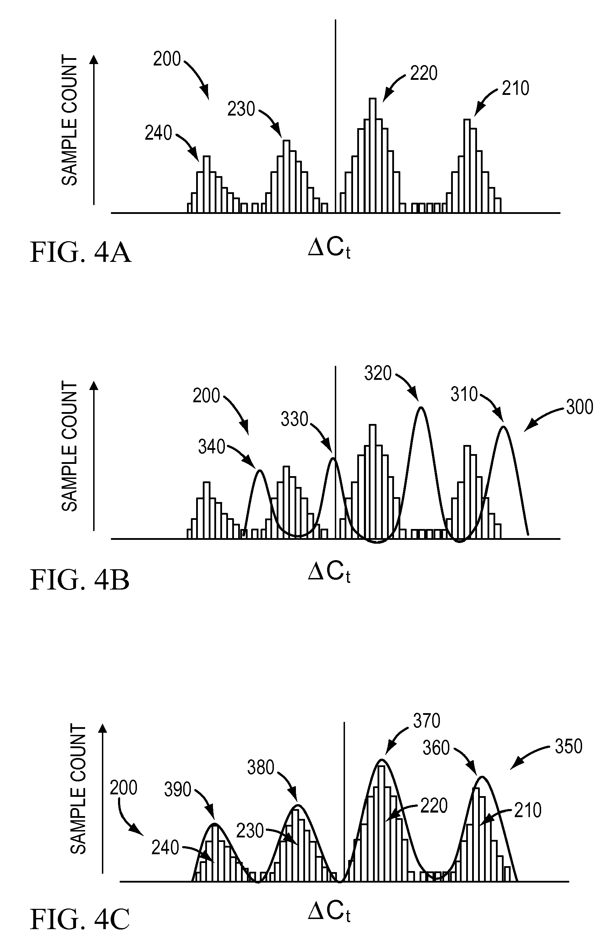

**FIGS. 4A -FIG. 4C** depict a step of assigning copy number for various embodiments of methods for the determination of gene copy number in a biological sample.

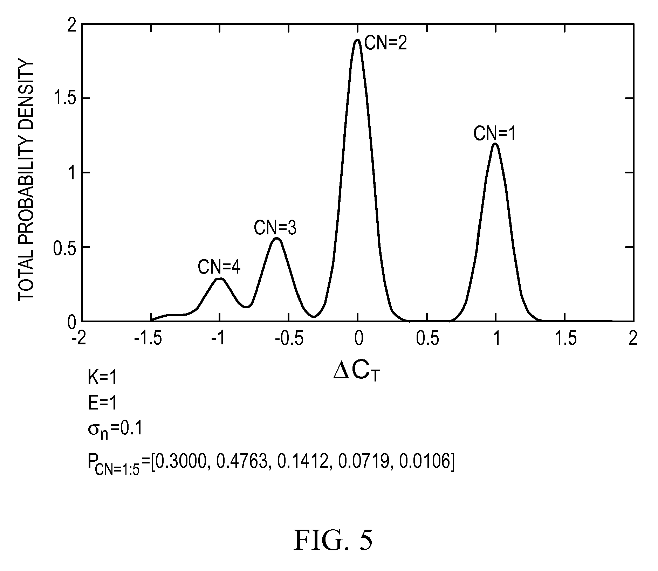

**FIG. 5** is an exemplary probability sample frequency population after various embodiments of methods for the determination of gene copy number, which was estimated using a probability density function model based on a normal distribution.

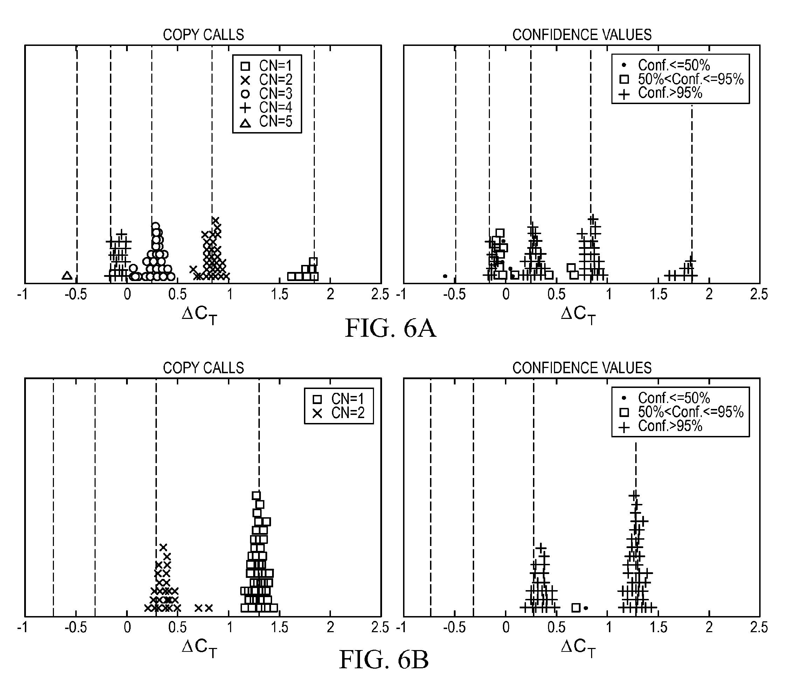

**FIG. 6A** and **FIG. 6B** are graphs that depict the confidence values of a copy number call for various embodiments of methods for the determination of the copy number related to probability density graphs for two sets of data having essential equivalent sample variances but different probability densities.

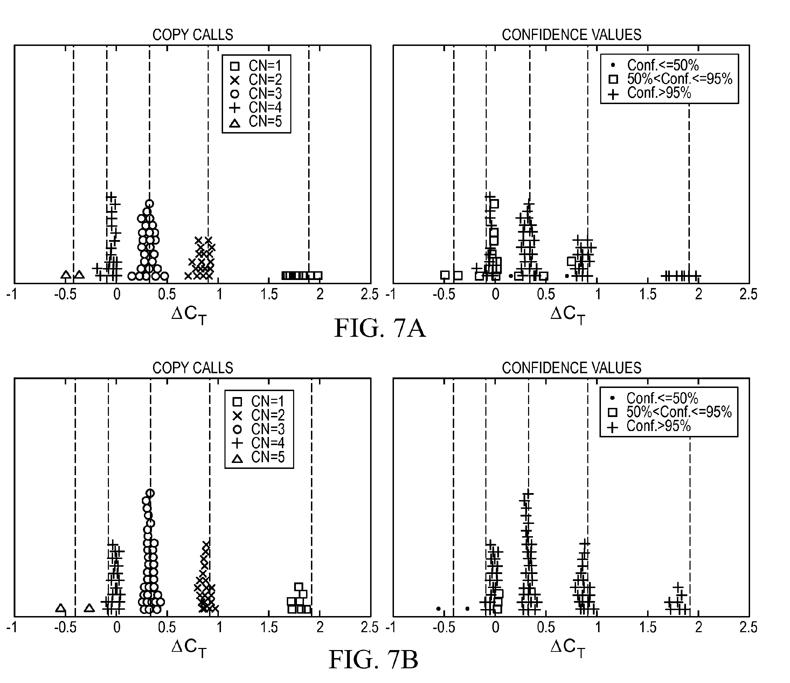

**FIG. 7A** and **FIG. 7B** are graphs that depict the confidence values of a copy number call for various embodiments of methods for the determination of the copy number related to probability density graphs for a set of copy number calls for two sample sets having the same probability densities but different sample variances.

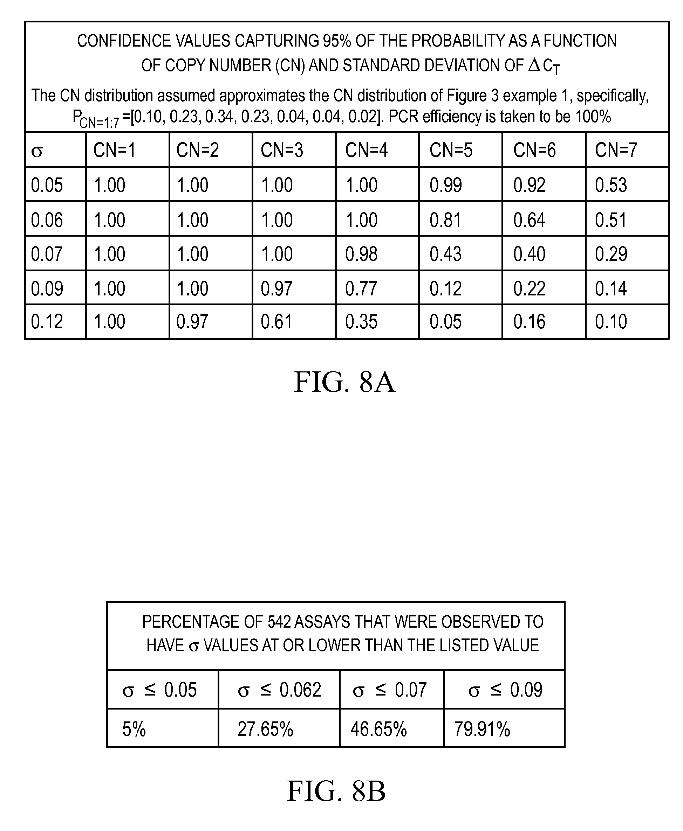

**FIG. 8A** is a table depicting the confidence values at 95% probability of a copy number call for various embodiments of methods for the determination of the copy number as a function of copy number and sample standard deviation.
**FIG. 8B** depicts the distribution of a plurality of sample determinations having targeted sample variances, as shown in **FIG. 8A.**

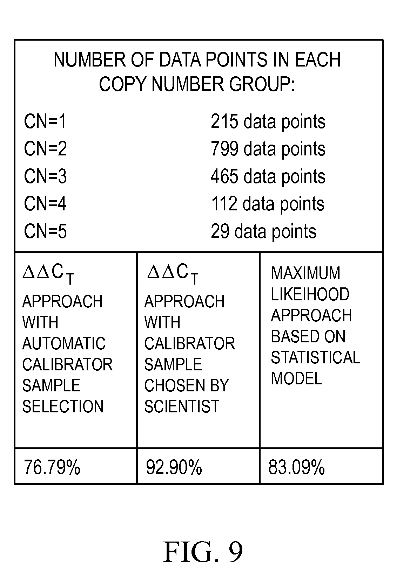

**FIG. 9** is a chart depicting the assignment of copy number for a plurality of samples using various embodiments of methods for the determination of the copy number in comparison to an established method for assignment of copy number.

## DETAILED DESCRIPTION

What is disclosed herein are various embodiments of methods for the determination of a copy number of a target genomic sequence; either a target gene or genomic sequence of interest, for each sample in a set of biological samples. In various embodiments, a model drawn from a probability density function (PDF) may be used as the basis for the assignment of a copy number of a target genomic sequence in a biological sample. The method is implemented on a computer and comprises:

calculating a $\Delta C_t$ value for a target genomic sequence for each sample in a set of biological samples using an endogenous reference genomic sequence, wherein $\Delta C_t$ is the difference in threshold cycles for the target and endogenous reference genomic sequence;
constructing a frequency distribution of the $\Delta C_t$ values calculated for each sample, wherein the frequency distribution comprises a set of collection of samples having similar $\Delta C_t$ values for a target genomic sequence (sub-distributions of $\Delta C_t$ values) for the target genomic sequence in a set of biological samples;
and determining a copy number for the target genomic sequence for each sample in the set of biological samples, wherein the determination is an assignment of a copy number for each sample based on a measure of fit between a probability density function model based on said sub-distributions of $\Delta C_t$ values and the frequency distribution.

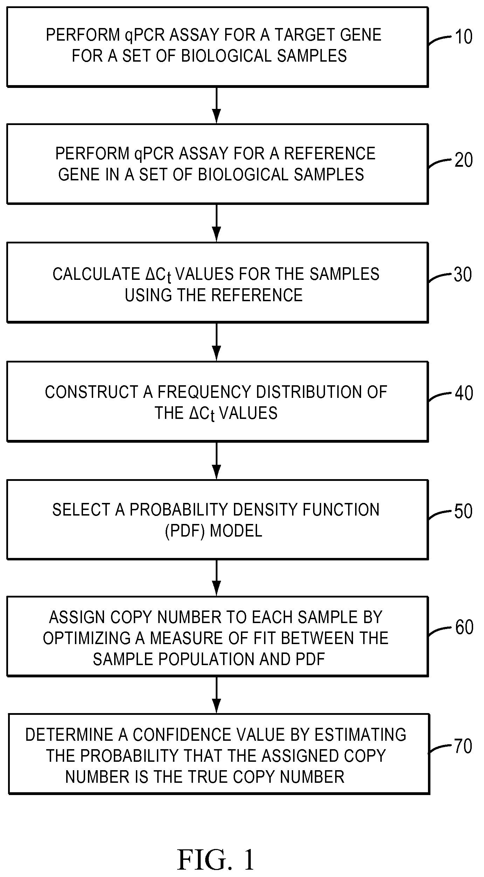

The type of assay that is used to provide the data for various embodiments of methods for the determination of a copy number is known to one of ordinary skill in the art as the real-time quantitative polymerase chain reaction (real-time qPCR). Though subsequent examples provided may utilize an assay format known as TaqMan®, various methods for the determination of a copy number may be used with any assay that provides quantitative data. For example, but not limited by, one of ordinary skill in the art would recognize Molecular Beacons, Amplifluor®* Primers, Scorpion™ Primers, Plexor™ Primers, and BHQ*plus*™ Probes as providing assay formats for qPCR. In that regard, any assay format that is a sequence-specific qPCR assay format may be used to provide data for various embodiments of methods for the determination of a copy number of a target genomic sequence in a biological sample.
According to steps **10** and **20** of **FIG. 1,** a qPCR assay for a target genomic sequence may be run on a plurality of samples, where each sample may be run in a plurality of replicates. For the determination of copy number using qPCR, run simultaneously with the samples, an endogenous reference genomic sequence; either an endogenous reference gene or endogenous reference genomic sequence, having a known number of copies is also assayed. It is desirable for the endogenous reference genomic sequence to have little observed variation in copy number for in the population from which the samples of interest are drawn. For example, the RNase P H1 RNA gene is known to exist in two copies in a human diploid gene.

To perform **10** and **20** of **FIG. 1,** various embodiments of an assay system as depicted in **FIG. 2** may be used. According to various embodiments of system 100, as shown in **FIG. 2,** a sample with a target genomic sequence can be loaded into a sample support device **20** of thermal cycler apparatus **50.** Various embodiments of a sample support device may have a plurality of sample regions. In various embodiments, sample support device **20** may be a glass or plastic slide with a plurality of sample regions **24,** which may be isolated from the ambient by cover **22.** Some examples of a sample support device may include, but are not limited by, a multi-well plate, such as a standard microtiter 96-well, a 384-well plate, or a microcard. The sample regions in various embodiments of a sample support device may include depressions, indentations, ridges, and combinations thereof, patterned in regular or irregular arrays on the surface of the substrate. As depicted in **FIG. 2,** a sample support device may be placed in a thermal cycler apparatus **50.** In various embodiments of a thermal cycler apparatus, there may be a heat block, **60,** and a detection system **51.** The detection system **51** may have an illumination source **52** that emits electromagnetic energy **56,** and a detector **54,** for receiving electromagnetic energy **57** from samples in sample support device **20.** Computer system **500** can control the function of the thermal cycler apparatus. Additionally, computer system **500** may provide data processing and report preparation functions. All such instrument control functions may be dedicated locally to the thermal cycler apparatus **50,** or computer system **500** may provide remote control of part or all of the control, analysis, and reporting functions, as will be discussed in more detail subsequently.

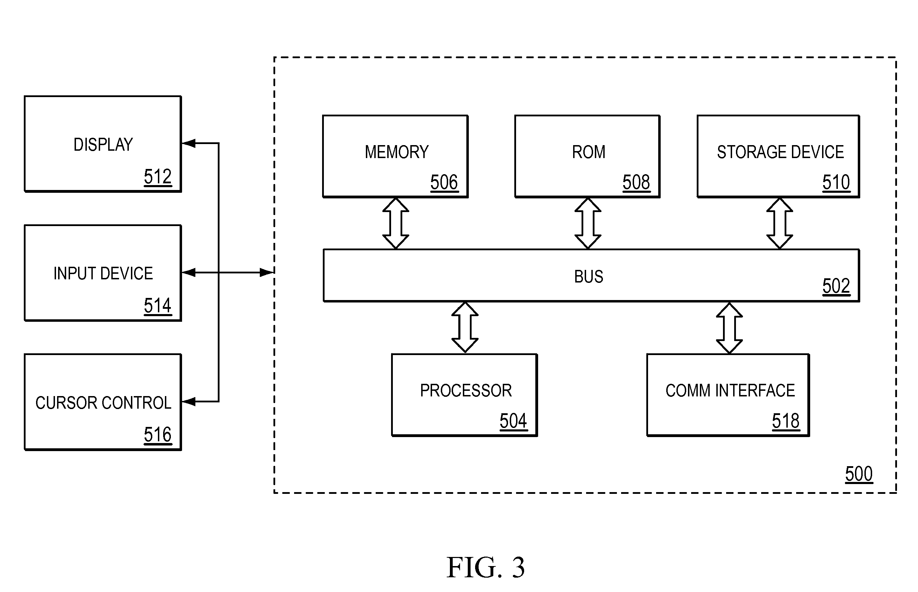

**FIG. 3** is a block diagram that illustrates a computer system **500,** according to various embodiments, upon which embodiments of methods for the analysis of PBA data may be implemented. Computer system **500** includes a bus **502** or other communication mechanism for communicating information, and a processor **504** coupled with bus **502** for processing information. Computer system **500** also includes a memory **506,** which can be a random access memory (RAM) or other dynamic storage device, coupled to bus **502** for determining base calls, and instructions to be executed by processor **504.** Memory **506** also may be used for storing temporary variables or other intermediate information during execution of instructions to be executed by processor **504.** Computer system **500** further includes a read only memory (ROM) **508** or other static storage device coupled to bus **502** for storing static information and instructions for processor **504.** A storage device **510,** such as a magnetic disk or optical disk, is provided and coupled to bus **502** for storing information and instructions.

Computer system **500** may be coupled via bus **502** to a display 512, such as, but not limited by, a cathode ray tube (CRT), liquid crystal display (LCD), or light-emitting diode (LED) for displaying information to a computer user. However, one of ordinary skill in the art may readily recognize that there are various ways of outputting data to an end user in a variety of forms, for example, but not limited by, having a report sent to a printer. An input device 514, including alphanumeric and other keys, is coupled to bus **502** for communicating information and command selections to processor **504.** Another type of user input device is cursor control **516,** such as a mouse, a trackball or cursor direction keys for communicating direction information and command selections to processor **504** and for controlling cursor movement on display **512.** This input device typically has two degrees of freedom in two axes, a first axis (e.g., x) and a second axis (e.g., y), that allows the device to specify positions in a plane. A computer system **500** provides base calls and provides a level of confidence for the various calls. Consistent with certain implementations of the invention, base calls and confidence values are provided by computer system **500** in response to processor **504** executing one or more sequences of one or more instructions contained in memory **506.** Such instructions may be read into memory 506 from another computer-readable medium, such as storage device **510.** Execution of the sequences of instructions contained in memory **506** causes processor **504** to perform the process states described herein. Alternatively hard-wired circuitry may be used in place of or in combination with software instructions to implement the invention. Thus implementations of the invention are not limited to any specific combination of hardware circuitry and software.

The term "computer-readable medium" as used herein refers to any media that participates in providing instructions to processor **504** for execution. Such a medium may take many forms, including but not limited to, non-volatile media, volatile media, and transmission media. Non-volatile media includes, for example, optical or magnetic disks, such as storage device 510. Volatile media includes dynamic memory, such as memory **506.** Transmission media includes coaxial cables, copper wire, and fiber optics, including the wires that comprise bus 502. Transmission media can also take the form of acoustic or light waves, such as those generated during radio-wave and infra-red data communications.

Common forms of computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, PROM, and EPROM, a FLASH-EPROM, any other memory chip or cartridge, a carrier wave as described hereinafter, or any other medium from which a computer can read.

Various forms of computer readable media may be involved in carrying one or more sequences of one or more instructions to processor **504** for execution. For example, the instructions may initially be carried on magnetic disk of a remote computer. The remote computer can load the instructions into its dynamic memory and send the instructions over a telephone line using a modem. A modem local to computer system **500** can receive the data on the telephone line and use an infra-red transmitter to convert the data to an infra-red signal. An infra-red detector coupled to bus **502** can receive the data carried in the infra-red signal and place the data on bus **502**. Bus **502** carries the data to memory **506,** from which processor **504** retrieves and executes the instructions. The instructions received by memory **506** may optionally be stored on storage device **510** either before or after execution by processor **504.**

As depicted in step **30** of **FIG. 1,** the $C_t$ values between the threshold cycles of the target and endogenous reference sequences are used to calculate $\Delta C_t$ each sample analyzed. According to various embodiments, the $\Delta C_t$ values are used as the basis for fit in the assignment of a copy number to a sample, as well as in the determination of a confidence value.

By way of providing an overview of the calculations for $\Delta C_t$ and $\Delta\Delta C_t$ for a copy number assay, the calculation of $\Delta C_t$ values from a data set is based on the equation for the progress of reaction for a PCR assay. It is well know that for a PCR reactions the equation describing the exponential amplification of PCR is given by:

$$(EQ.\ 1) \qquad X_n = X_o\,[(1 + E_X\,)^n]$$

where:

$X_n$ = the number of target molecules at cycle n
$X_o$ = the initial number of target molecules
$E_x$ = the efficiency of the target amplification
n = the number of cycles

from that relationship, the concentration of a genomic sequence at the threshold is:

$$(EQ.\ 2) \qquad X_{Ct,x} = X_o\,[(1 + E_X\,)^{Ct,x} = K_X$$

where:

$X_{Ct,x}$ = the number or target molecules at $C_t$
$X_o$ = the initial number of target molecules
$E_x$ = the efficiency of the target amplification
$C_{t,x}$ = the number of cycles at $C_t$
$K_x$ = a constant

From this it is evident that for a target genomic sequence; either a gene or genomic sequence of interest, the concentration of target formed in the reaction at $C_t$ is a constant K, and therefore characteristic of the reaction. Generally, K may vary for various target genomic sequences, due to a number of reaction variables, such as, for example the reporter dye used in a probe, the efficiency of the probe cleavage, and the setting of the detection threshold. Additionally, as previously described, is generally held that the assumption that the efficiencies of reactions are optimized and essentially the same. Under such conditions and assumptions, it can be shown through the algebraic manipulation of EQ. 2, that normalizing a target genomic sequence of interest of to an endogenous reference reaction at $C_t$ yields the following relationship:

$$(EQ.\ 3) \qquad X_N = K\,[(1 + E)^{-\Delta C_t}]$$

where:

$X_N$ = is the normalized amount of the target
$\Delta C_t$ = is the difference in threshold cycles for the target and endogenous reference genomic sequence

Further, it should be noted that for the comparative $C_t$ method, or $\Delta\Delta C_t$ method, that the relative concentration of a target genomic sequence to a calibrator is:

$$(EQ.\ 4) \qquad X_{N,t}/\,X_{N,c} = (1 + E)^{-\Delta\Delta C_t}$$

where:

$X_{N,t}/ X_{N,c}$ = is the relative concentration of the target relative to the calibrator; and
$\Delta\Delta C_t$ = is the normalized difference in threshold cycles for the target and the calibrator

Then, as previously mentioned, as assays are optimized to ensure a maximum in the reaction efficiency, or an efficiency of 1, then EQ. 4 simplifies to the calculation known to one of ordinary skill in the art for the comparative $C_t$ method previously given:

$$(EQ. 5) \qquad X_{N,t}/ X_{N,c} = 2^{-\Delta\Delta C_t}$$

For various embodiments of methods for the determination of a copy number disclosed herein, a first step toward assigning a copy number to a sample may include the construction a frequency distribution of $\Delta C_t$ values for a plurality of samples having different copy numbers of a target genomic, as depicted in steps **40** of **FIG. 1.** In that regard, in **FIG. 3A,** an example is shown for a frequency distribution of $\Delta C_t$ values for a hypothetical sample population. The sample frequency distribution **200** may include distinct sample sub-distributions, such as sample sub-distributions **210, 220, 230,** and **240** of **FIG. 4A,** where each distinct sub-distribution corresponds to a sub-distribution **210, 220, 230,** and **240** that is a collection of samples having similar $\Delta C_t$ values for a target genomic sequence. As will be discussed in more detail subsequently, such sub-distributions may be described by parameters, such as, but not limited by a sub-distribution mean and a sub-distribution variance.

According to various embodiments, as depicted in step **50** of **FIG. 1,** a probability density function (PDF) is selected as the basis for the assignment of a copy number of a target genomic sequence in a biological sample. As previously discussed, in various embodiments, parameters defining the selected PDF model are searched until the values for the parameters that define the PDF model are optimized to a measure of fit between the observed sample frequency distribution and the PDF model. Though a normal distribution is a probability distribution function by which many naturally occurring populations may be described, as well as being a model that is generally well-understood, any type of mono-modal distribution may be selected as appropriate for a particular observed sample frequency distribution. Accordingly, for the purpose of illustration, the normal distribution will be used in examples given subsequently. However, in addition to the normal distribution, other model monomodal distributions useful as candidates may include, but are not limited by, the Burr, Cauchy, Laplace, and logistic distributions.

According to various embodiments, an equation for copy number as a function of $\Delta C_t$ data generated from qPCR assays having a monomodal PDF sub-distribution for each copy number cn with mean, $\mu_{\Delta Ct}(cn)$, is constrained to be described as:

$$(EQ. 6) \qquad \mu_{\Delta Ct}(cn) = K - \log_{(1+E)}(cn)$$

where:

$\mu_{\Delta Ct}(cn)$= is the mean of the $\Delta C_t$ sub-distributions as a function of copy number; where cn is a non-zero positive integer
K = is a constant; and
log $(1 + E)$ (cn) = the log to the base $(1 +E)$ of copy number cn where E is the efficiency of the PCR amplification of the gene of interest as a result of EQ. 2, where, as previously described, variation in $\Delta C_t$ data around $\mu_{\Delta Ct}(cn)$ may arise within and between samples with the same copy number due to various factors such as, for example, but not limited by, thermal fluctuations in the thermal cycler, and binding behaviors of PCR primers and probes. In various embodiments, an exemplary PDF model may be a normal distribution and, in this case, the full PDF model can be directly characterized by $\mu_{\Delta Ct}(cn)$, K, E, the sample variance, $\sigma$, and the probability of each copy number. Though these parameters directly characterize a PDF using the exemplary normal distribution, it should be understood, as previously mentioned, that any mono-modal distribution PDF may be used so long as the mean of the PDF is constrained to follow EQ.6. Accordingly, it should be understood that various mono-modal PDFs, such as, but not limited by, the normal, the Burr, Cauchy, Laplace, and logistic distributions may have different sets of parameters that characterize such model PDF distributions.

According to various embodiments, as depicted in step **60** of **FIG. 1,** each sample having a $\Delta C_t$ for a target genomic sequence used to construct a frequency distribution may be assigned a copy number for that target genomic sequence based on optimizing a measure of fit between the probability density function model and the sample frequency distribution, as depicted in **FIG. 4B** and **FIG. 4C**. In **FIG. 4B,** model distribution **300,** including sub-distributions **310, 320, 330,** and **340,** represents one of a plurality of distributions constructed by varying parameters characterizing a PDF model as selected in **50** of **FIG. 1**. In **FIG. 4C,** model distribution **350,** including sub-distributions **360, 370, 380,** and **390,** represents still another of a plurality of distributions constructed by varying parameters characterizing the selected PDF model.

In various embodiments, a parameter space of parameters defining a PDF model as selected in **50** of **FIG. 1** may be searched to generate a plurality of model distributions such as model distributions **300** and **350** of **FIG. 4B** and **FIG. 4C,** respectively. In various embodiments, a metric of merit may be selected to optimize the measure of fit between a PDF model distribution, such as model distributions **300** and **350** of **FIG. 4B** and **FIG. 4C,** respectively. Various embodiments may have sub-distributions described, for example, by parameters $\mu_{\Delta Ct}(cn)$, K, E, $\sigma$, and copy number probabilities $\Pi_{cn}$, which directly characterize a PDF based on normal sub-distributions. According to various embodiments, the parameter space may be searched using techniques, such as, but not limited by, grid searching, gradient searching, conjugate gradient searching, simulated annealing searching, genetic algorithms searching, and non-linear least squares searching. In various embodiments, the search may be initialized with a prescribed number of copy numbers associated with non-zero probability that may or may not be revised during the search. In various embodiments, the probabilities for each copy number may be initialized with all non-zero probabilities set to the same value or any other prescribed distribution and these probabilities may be revised during the search. In various embodiments, the metric of merit selected to evaluate the fit of the model PDF to the sample frequency distribution may be derived from, for example, but not limited by, the entropy (information theoretic) figure of fit, the squared differences figure of fit, and probability density figure of fit. In various embodiments using the squared difference figure of fit, the metric of merit for evaluating fit is based on a minimum value of the squared differences between the sample sub-distributions and the model PDF. For various embodiments using the entropy or probability density metrics are based on maximizing the sum of these measures across the samples evaluated with the candidate model PDF. Once the parameters that define the model PDF are determined through this process, copy numbers may be assigned to the sample sub-distributions, and hence to the samples comprising the sub-distributions.

According to various embodiments, for a calculated model based on a PDF model as selected in **50** of **FIG. 1,** for example as depicted in **300 FIG. 4B,** and defined by a first set of parameters $K_1$, $E_1$, $\sigma_1$, and $\Pi_{cn,1}$ the assumption may be made that $\sigma_1$ is identical for all sub-distributions **310, 320, 330,** and **340** in calculated model **300.** Similarly, for any subsequent calculated model, for example as depicted in **350 FIG. 4C,** and defined by a second set of parameters $K_2$, $E_2$, $\sigma_2$, and $\Pi_{cn,2}$ then $\sigma_2$ may be identical for all sub-distributions **360, 370, 380,** and **390** in calculated model **350.** In various embodiments, the parameter space of parameters, for example such as K, E, $\sigma$, and $\Pi_{cn}$ for a PDF model defined by EQ. 5, may be searched using a grid search. For various embodiments, a metric of merit, such as maximizing the probability of the observed sample frequency distribution against the PDF model distribution, may be used to asses the fit between population **200** and the calculated models **300** and **350** of **FIG. 4B** and **FIG. 4C,** respectively. For the calculated model **300** of **FIG. 4B,** it is apparent in this representation that the fit of the sample population **200** to the calculated model **300,** constructed using a selection of a first set of parameters $K_1$, $E_1$, $\sigma_1$, and $\Pi_{cn,1}$ is not good. For various embodiments, when the process is complete, a fit is then indicated by the metric of merit selected. For example, the fit of the sample population **200** to the calculated model **350** is a good fit. In such a case, the parameters of the best fit model are parameters that also estimate the sample frequency distribution. For the example shown in **FIG. 4C,** the second set of parameters $K_2$, $E_2$, $\sigma_2$, and $\Pi_{cn,2}$ used to calculate model **350** are also parameters that estimate the sample frequency distribution. According to various embodiments, such parameters may be used to assign a copy number to sub-distributions such as sample sub-distributions **210, 220, 230,** and **240** of sample distribution **200** depicted in **FIGS. 4A-4C.**

**FIG. 5** shows an exemplary determination of copy number for a set of samples using various embodiments of statistical method. The PDF shown **FIG. 5** was estimated from a set of 32 plates that contained data from 250 individuals and four different genes from the C4 region of the genome. As is apparent from inspection of **FIG. 5,** the PDF for this sample set covers the range from copy numbers (CNs) 1 to 5. The assumption of normality for the individual sub-distributions was tested by examining the sub-distribution of the samples for the sub-distribution at CN=2, which represented a set of 542 assays. To test the assumption of normality, Lilliefors test was used, with the results that 71.8% of the members of samples for the sub-distribution at CN=2 pass the test. The test of normality may not meaningful for higher CN values in the exemplary PDF of **FIG. 5** because of insufficient data. A characteristic of the exemplary distribution of **FIG. 5,** which is exemplary of various methods utilizing the fit to a PDF, is that the separation between the $\mu CN$'s decrease as CN increases. This is a direct consequence of the logarithmic relationship between $\Delta C_t$ and the concentration of genomic material within the context of PCR. As a result of the decreasing separation between sub-distributions with increasing CN, the variability of $\Delta C_t$ values has a larger impact on the resolution of the higher CN values. As measurement variability increases, the average confidence of higher CN calls will decrease much faster than confidence values for lower CN's. Additionally, as will be discussed in more detail subsequently, the relative probability of a copy number, the $P_{CN}$, can influence the confidence value associated with a call. An approximate trend is that the confidence of copy calls increases with increases in the frequency of samples belonging to that CN group. Various embodiments may be used to specify optimum $\Delta C_t$ decision boundaries for CN value assignment. As is depicted in **FIG. 5,** it is apparent that these boundaries should be placed at the minimum PDF values between the peaks of the PDF since, to either side of these boundaries there is a larger likelihood that the CN corresponds to that of the closer peak in the PDF.

As depicted In **FIG. 1,** step **70,** after the set of sample sub-distribution populations included in the sample frequency distribution have copy numbers assigned, thereby assigning copy numbers to every sample included in each sample sub-distribution, a confidence value for every sample in the sample frequency distribution may be determined.

According to various embodiments, the confidence that the assigned copy number is the true copy number within the assumption that the PDF model is accurate may be described most generally by the probability that this is so as described in the following equation:

$$(EQ.\ 6) \qquad P(cn_{assigned} = cn_{true}) = P(cn_{assigned}|\ \Delta C_r\text{'s})$$

$$= P(\Delta Ct_r\text{'s} \mid cn_{assigned})P(cn_{assigned}) / P(\Delta Ct_r\text{'s})$$

$$= \frac{\Pi_{cn_{assigned}} F(\Delta Ct_r\text{'s}; cn_{assigned})}{\sum_{cn} \Pi_{cn} F(\Delta Ct_r\text{'s}; cn)} \quad \text{where}$$

$\Delta Ct_r$'s refers to the replicate observations for a given person, and F is the probability distribution function chosen for the sub-distributions that is constrained by requiring that its mean is given by:

$\mu_{cn} = K\text{-}\log_{(1+E)} (cn)$

$\Pi cn$ is the probability of copy number cn

As exemplary, for various embodiments where F is assumed to be a normal distribution, analyses taken from mathematical statistics can be used to produce the following:

$$(EQ.8) \qquad P(cn_{assigned} = cn_{true}) = \left[ 1 + \sum_{cn \neq cn_a} \frac{\Pi cn}{\Pi cn_a} e^{-\Omega} \right]^{-1}$$

where subscript a is shorthand for *assigned* $\Pi_{cn}$ is the probability of copy number cn

$$\Omega \equiv \frac{1}{\sigma^2} \log_{(1+E)} (\frac{cn}{cn_a}) \left( (\hat{\mu}_r - K) + \frac{\log_{(1+E)} (cn_a cn)}{2} \right)$$

$$\hat{\mu}_r = \frac{1}{N_r} \sum_{\substack{all\ replicates \\ for\ a\ person}} \Delta Ct_r\ ;$$

and $\sigma^2$ = the variance of the sub-distributions for each copy number.

According to various embodiments, a confidence value may be determined by first identifying the two sample sub-distributions having the greatest number of samples, and determine the sub-distribution means for the two populations. Such a mean would be the mean of replicate means, or the mean of $\hat{\mu}_r$ given above in EQ. 7. Recalling EQ. 6:

$$(EQ.\ 6) \qquad \mu_{\Delta Ct}(cn) = K\text{-}\log_{(1+E)} (cn)$$

where:

$\mu_{\Delta Ct}$(cn)= is the mean of a of the $\Delta C_t$ sub-distributions as a function of copy number; where cn is a non-zero integer
K = is a constant; and
$\log_{(1+E)}$ (cn) = the log to the base (1 +E) of the copy number of a gene in a sub-distribution of sample distributions, where E is the efficiency of the PCR amplification

Then, for various embodiments, $\mu_{\Delta Ct}$(cn) is estimated for the two populations having the greatest number of samples, yielding two independent equations, which may be used to solve for the two unknowns, K and E. Additionally, the variance for the mean of sample means, $\sigma_{msm}$ may be determined, as well as $\Pi_{cn}$ the probability of copy number cn. In various embodiments, a distribution of probabilities that the assigned copy number is the true copy number may be generated using the parameters K, E, $\sigma_{msm}$, and $\Pi_{cn}$. According to various embodiments, a Bootstrap technique may be used to generate such a distribution. In various embodiments, once the distribution of the probability measure given by EQ. 7 using the Bootstrap technique is generated, then a confidence level may be selected for the EQ. 7 probability measure. For example, in various embodiments a confidence level assuring that there is a 95% chance that the EQ. 7 probability is equal to or higher than the value determined for this quantity. As will be discussed in more detail subsequently, variables such as the number of samples comprising a sub-population, the copy number, and sample variance may all impact the degree to which high values for the EQ. 7 probability can be achieved.

The set of figures represented by **FIGS. 6A** and **6B;** and **FIGS. 7A** and **7B** demonstrate the various embodiments of methods for the determination of a copy number for a genomic sequence disclosed herein. The frequency distributions represented in **FIGS. 6A** and **6B;** and **FIGS. 7A** and **7B** are results from 93 samples run through four different assays that target the C4 regions of the genome. For each sample, the mean $\Delta C_t$ value across replicates is shown using a resolution of 0.05 $\Delta C_t$ units. The height assigned to a sample has no particular significance for the sample but, over the population, the final height of points within a 0.05 $\Delta C_t$ interval is an approximation of the relative frequency of samples that fall within that interval. The blue vertical lines show the positions of $\mu_{CN}$ according to the statistical model. For each data set by **FIGS. 6A** and **6B;** and **FIGS. 7A** and **7B,** there are two panels. In the left panel, the CN assignments are shown, while in the right panel, the confidence values are represented. The standard deviations shown above the plots are estimated from the data shown.

According to various embodiments, both copy number and sample variance may have an impact on the determination of a copy number, which is evident from the inspection of **FIGS. 6A** and **6B;** and **FIGS. 7A** and **7B.** The populations represented in **FIGS. 6A** and **6B** have similar variance; where the variance for **FIG. 6A** is 0.086, and the variance for **FIG. 6B** is 0.090, but their CN distributions differ. The sample frequency distribution represented in **FIG. 6B** has fewer high CN samples and more points are called with high confidence. For the comparison of the sample frequency distributions represented by **FIGS. 7A** and **7B,** while both sample frequency distributions have similar CN distributions but variances differ. The sample frequency distribution of **FIG. 7A** has a sample variance (0.071) that is higher than the sample variance of sample frequency distribution of **FIG. 7B** (0.062). As the sample frequency distribution represented by **FIG. 7B** has the lower variance, it additionally has more points called with high confidence.

Further illustrate the impact of copy number and variance on the determination of copy number using various embodiments is illustrated in the tables presented in **FIG. 8A** and **FIG. 8B.** The illustrated in **FIGS. 6A** and **6B;** and **FIGS. 7A** and **7B** demonstrate that copy numbers up to 4 can be detected with high confidence if experimental conditions can be controlled to achieve $\sigma$= 0.062, such as shown in **FIG. 7B.** Additionally, as illustrated in the results presented in **FIG. 7A,** up to 3 copy numbers can be detected with $\sigma$=0.071. In support of such results, the theoretical results presented in **FIG. 7A** based on various embodiments of the statistical model appear to be consistent with these observations. **FIG. 8A** illustrates the confidence value that can be expected for 95% or more of the samples as a function of copy number (CN) and $\sigma$, according to various embodiments. The copy number distribution used in the computations is similar to the distribution of the experimental data shown in **FIG. 7B.** For the theoretical results presented in **FIG. 8A,** for $\sigma$ = 0.06, the computations suggest that up to four copies can be detected with a high level of confidence. At $\sigma$ = 0.05, the theoretical results suggest that it is possible to detect 6-copy samples with a high level of confidence. **FIG. 8B** provides some empirical evidence that for various embodiments of a statistical model for the determination of a copy number, it is practical to achieve these resolution levels: In **FIG. 8B,** the percentage of assays achieving the listed values for $\sigma$ is presented. Combining the data shown in **FIGS. 8A** and **8B,** it is clear that for various embodiments of a statistical model for the determination of a copy number, most assays should be able to resolve CN=3, many can resolve CN=4, and it is possible to resolve CN=6.

In **FIG. 9,** the comparison of various embodiments of a statistical model for the determination of a copy number to the $\Delta\Delta C_t$ approach is shown. The data used for the comparison was generated using a set of 200 assays with 7 to 37 aneuploidy samples per plate; hence, the copy number value for each sample was known. These data were analyzed in three ways. The first analysis was the $\Delta\Delta C_t$ approach using the median $\Delta C_t$ value over the data set as the calibrator value with the assumption that it corresponds to two copies of the target gene. The second analysis was the $\Delta\Delta C_t$ approach using a sample assigned by a scientist as the calibrator; the CN for this sample was also assigned by the scientist. The third analysis assigned CN values by an embodiment of a statistical model for the determination of a copy number, wherein the embodiment requires pre-specification of the CN value that is expected to occur most frequently. **FIG. 9** shows the results of these analyses.

In addition to the steps of various embodiments shown in **FIG. 1,** one of ordinary skill in the art would recognize additional steps that may be routinely performed on qPCR data, for example, but not limited by, the steps of validating and filtering the data, as well as excluding outliers. In various embodiments, detecting outliers from replicates is performed by assuming that the variation of replicates about the replicate mean is the same for all samples, estimating this variation using data from all samples excluding replicates that deviate from the replicate median by more than a selected amount, and labeling a replicate as an outlier if it differs from the replicate median by more than a selected number of standard deviations. According to various embodiments, the selection may be done based on the square root of the variance. As demonstrated above in **FIGS. 6A** and **6B; FIGS. 7A** and **7B,** and **FIGS. 8A** and **8B,** the sample variance may have a significant impact on results for determining the copy number of target genomic sequence; either a gene or genomic sequence of interest. In various embodiments, the exclusion of verified outliers may then provide additional robustness to the copy number determination.

While the principles of this invention have been described in connection with specific embodiments, it should be understood clearly that these descriptions are made only by way of example and are not intended to limit the scope of the invention. What has been disclosed herein has been provided for the purposes of illustration and description. It is not intended to be exhaustive or to limit what is disclosed to the precise forms described. Many modifications and variations will be apparent to the practitioner skilled in the art. What is disclosed was chosen and described in order to best explain the principles and practical application of the disclosed embodiments of the art described, thereby enabling others skilled in the art to understand the various embodiments and various modifications that are suited to the particular use contemplated. It is intended that the scope of what is disclosed be defined by the following claims and their equivalence.

## Claims

1. A method for determining the copy number of a genomic sequence for each sample in a set of biological samples, the method comprising:

   calculating a $\Delta C_t$ value for a target genomic sequence for each sample in a set of biological samples using an endogenous reference genomic sequence, wherein $\Delta C_t$ is the difference in threshold cycles for the target and endogenous reference genomic sequence;
   constructing a frequency distribution of the $\Delta C_t$ values calculated for each sample, wherein the frequency distribution comprises a set of collection of samples having similar $\Delta C_t$ values for a target genomic sequence (sub-distributions of $\Delta C_t$ values) for the target genomic sequence in a set of biological samples;
   and determining a copy number for the target genomic sequence for each sample in the set of biological samples, wherein the determination is an assignment of a copy number for each sample based on a measure of fit between a probability density function model based on said sub-distributions of $\Delta C_t$ values and the frequency distribution,
   wherein the method is implemented on a computer.

2. The method of Claim 1, wherein the method further comprises calculating a confidence value for the copy number determined for the first target genomic sequence for each sample in the set of biological samples.

3. The method of Claim 2, wherein the confidence value is an estimate of the probability that the assigned copy number for the target genomic sequence of each sample is the correct copy number based on the probability density function model at a designated confidence level.

4. The method of Claim 1, wherein the probability density function model is a monomodal distribution model.

5. The method of Claim 4, wherein the probability density function model is a normal distribution model.

6. The method of Claim 4, wherein the probability density function model is selected from Burr, Cauchy, Laplace, and logistic distribution models.

7. The method of Claim 1, wherein the method further comprises filtering the data to exclude outliers.

8. A computer program product comprising:

a computer-readable medium and computer-readable code embodied on said computer-readable medium which when run on a computer performs a method according to claims 1-7.

**Patentansprüche**

1. Verfahren zum Bestimmen der Kopienzahl einer genomischen Sequenz für jede Probe in einem Satz biologischer Proben, wobei das Verfahren Folgendes umfasst:

Berechnen eines ΔCt-Wertes für eine genomische Zielsequenz für jede Probe in einem Satz biologischer Proben unter Verwendung einer endogenen genomischen Referenzsequenz, wobei $\Delta C_t$ die Differenz in Grenzzyklen für die Zielsequenz und die endogene genomische Referenzsequenz ist;
Konstruieren einer Häufigkeitsverteilung der für jede Probe berechneten $\Delta C_t$-Werte, wobei die Häufigkeitsverteilung einen Satz einer Zusammenstellung von Proben mit ähnlichen $\Delta C_t$-Werten für eine genomische Zielsequenz (Unterverteilungen von $\Delta C_t$-Werten) für die genomische Zielsequenz in einem Satz biologischer Proben umfasst;
und Bestimmen einer Kopienzahl für die genomische Zielsequenz für jede Probe in dem Satz von biologischen Proben, wobei die Bestimmung eine Zuordnung einer Kopienzahl für jede Probe basierend auf einem Anpassungsmaß zwischen einem Modell der Wahrscheinlichkeitsdichtefunktion, basierend auf den Unterverteilungen der $\Delta C_t$-Werte, und der Häufigkeitsverteilung ist, wobei das Verfahren auf einem Computer implementiert wird.

2. Verfahren nach Anspruch 1, wobei das Verfahren ferner das Berechnen eines Konfidenzwertes für die Kopienzahl umfasst, die für die erste genomische Zielsequenz für jede Probe in dem Satz biologischer Proben bestimmt wurde.

3. Verfahren nach Anspruch 2, wobei der Konfidenzwert eine Abschätzung der Wahrscheinlichkeit ist, dass die zugeteilte Kopienzahl für die genomische Zielsequenz jeder Probe basierend auf dem Modell der Wahrscheinlichkeitsdichtefunktion im zugeordneten Konfidenzintervall die richtige Kopienzahl ist.

4. Verfahren nach Anspruch 1, wobei das Modell der Wahrscheinlichkeitsdichtefunktion ein monomodales Verteilungsmodell ist.

5. Verfahren nach Anspruch 4, wobei das Modell der Wahrscheinlichkeitsdichtfunktion ein Normalverteilungsmodell ist.

6. Verfahren nach Anspruch 4, wobei das Modell der Wahrscheinlichkeitsdichtefunktion aus Burr-, Cauchy-, Laplace- und logistischen Verteilungsmodellen ausgewählt ist.

7. Verfahren nach Anspruch 1, wobei das Verfahren ferner das Filtern der Daten zum Ausschluss von Ausreißern umfasst.

8. Computerprogramm-Produkt, umfassend:

ein computerlesbares Medium und einen computerlesbaren Code, ausgeführt auf dem computerlesbaren Medium, das beim Laufen auf einem Computer ein Verfahren nach den Ansprüchen 1 bis 7 ausführt.

**Revendications**

1. Procédé de détermination du nombre de copies d'une séquence génomique pour chaque échantillon dans un jeu d'échantillons biologiques, le procédé comprenant :

le calcul d'une valeur $\Delta C_t$ pour une séquence génomique cible pour chaque échantillon dans un jeu d'échantillons biologiques en utilisant une séquence génomique endogène de référence, où $\Delta C_t$ est la différence de cycles de seuil entre la séquence génomique cible et la séquence génomique de référence ;
la constitution d'une distribution de fréquences des valeurs $\Delta C_t$ calculées pour chaque échantillon, où la distribution de fréquences comprend un jeu de collections d'échantillons ayant des valeurs $\Delta C_t$ similaires pour une séquence génomique cible (sous-distributions de valeurs $\Delta C_t$) dans un jeu d'échantillons biologiques ; et
la détermination d'un nombre de copies pour la séquence génomique cible dans chaque échantillon du jeu d'échantillons biologiques, la détermination étant une affectation d'un nombre de copies pour chaque échantillon sur la base d'une mesure d'ajustement entre un modèle de fonction de densité de probabilité basé sur lesdites sous-distributions de valeurs $\Delta C_t$ et la distribution de fréquences,
dans lequel le procédé est mis en oeuvre par un ordinateur.

2. Procédé selon la revendication 1, comprenant en outre le calcul d'un intervalle de confiance pour le nombre de copies déterminé pour la première séquence génomique cible pour chaque échantillon dans le jeu d'échantillons biologiques.

3. Procédé selon la revendication 2, dans lequel la valeur de confiance est une estimation de la probabilité que le nombre de copies affecté à la séquence génomique cible de chaque échantillon soit le nombre exact de copies, sur la base du modèle de fonction de densité de probabilité à son niveau de confiance désigné.

4. Procédé selon la revendication 1, dans lequel le modèle de fonction de densité de probabilité est un modèle à distribution monomodale.

5. Procédé selon la revendication 4, dans lequel le modèle de fonction de densité de probabilité est un modèle à distribution normale.

6. Procédé selon la revendication 4, dans lequel le modèle de fonction de densité de probabilité est choisi parmi les modèles de distribution de Burr, Cauchy, Laplace et logistique.

7. Procédé selon la revendication 1, comprenant en outre le filtrage des données pour exclure les valeurs aberrantes.

8. Produit logiciel informatique comprenant :

un médium lisible par un ordinateur et un code lisible par un ordinateur incorporé sur ledit médium lisible par un ordinateur qui, utilisé par un ordinateur, effectue un procédé selon l'une quelconque des revendications 1 à 7.

```
┌─────────────────────────────────────────────┐
│   PERFORM qPCR ASSAY FOR A TARGET GENE        │─── 10
│    FOR A SET OF BIOLOGICAL SAMPLES            │
└─────────────────────────────────────────────┘
                     │
                     ▼
┌─────────────────────────────────────────────┐
│    PERFORM qPCR ASSAY FOR A REFERENCE         │─── 20
│   GENE IN A SET OF BIOLOGICAL SAMPLES         │
└─────────────────────────────────────────────┘
                     │
                     ▼
┌─────────────────────────────────────────────┐
│   CALCULATE ΔCt VALUES FOR THE SAMPLES        │─── 30
│          USING THE REFERENCE                  │
└─────────────────────────────────────────────┘
                     │
                     ▼
┌─────────────────────────────────────────────┐
│   CONSTRUCT A FREQUENCY DISTRIBUTION OF       │─── 40
│            THE ΔCt VALUES                     │
└─────────────────────────────────────────────┘
                     │
                     ▼
┌─────────────────────────────────────────────┐
│   SELECT A PROBABILITY DENSITY FUNCTION       │─── 50
│             (PDF) MODEL                       │
└─────────────────────────────────────────────┘
                     │
                     ▼
┌─────────────────────────────────────────────┐
│   ASSIGN COPY NUMBER TO EACH SAMPLE BY        │─── 60
│  OPTIMIZING A MEASURE OF FIT BETWEEN THE      │
│        SAMPLE POPULATION AND PDF              │
└─────────────────────────────────────────────┘
                     │
                     ▼
┌─────────────────────────────────────────────┐
│  DETERMINE A CONFIDENCE VALUE BY ESTIMATING   │─── 70
│   THE PROBABILITY THAT THE ASSIGNED COPY      │
│    NUMBER IS THE TRUE COPY NUMBER             │
└─────────────────────────────────────────────┘
```

FIG. 1

FIG. 2

EP 2 406 400 B1

FIG. 3

FIG. 4A

FIG. 4B

FIG. 4C

K=1
E=1
$\sigma_n$=0.1
$P_{CN=1:5}$=[0.3000, 0.4763, 0.1412, 0.0719, 0.0106]

FIG. 5

FIG. 6A

FIG. 6B

| CONFIDENCE VALUES CAPTURING 95% OF THE PROBABILITY AS A FUNCTION OF COPY NUMBER (CN) AND STANDARD DEVIATION OF $\Delta C_T$ | | | | | | | |
|---|---|---|---|---|---|---|---|
| The CN distribution assumed approximates the CN distribution of Figure 3 example 1, specifically, $P_{CN=1:7}$ =[0.10, 0.23, 0.34, 0.23, 0.04, 0.04, 0.02]. PCR efficiency is taken to be 100% | | | | | | | |
| $\sigma$ | CN=1 | CN=2 | CN=3 | CN=4 | CN=5 | CN=6 | CN=7 |
| 0.05 | 1.00 | 1.00 | 1.00 | 1.00 | 0.99 | 0.92 | 0.53 |
| 0.06 | 1.00 | 1.00 | 1.00 | 1.00 | 0.81 | 0.64 | 0.51 |
| 0.07 | 1.00 | 1.00 | 1.00 | 0.98 | 0.43 | 0.40 | 0.29 |
| 0.09 | 1.00 | 1.00 | 0.97 | 0.77 | 0.12 | 0.22 | 0.14 |
| 0.12 | 1.00 | 0.97 | 0.61 | 0.35 | 0.05 | 0.16 | 0.10 |

## FIG. 8A

| PERCENTAGE OF 542 ASSAYS THAT WERE OBSERVED TO HAVE $\sigma$ VALUES AT OR LOWER THAN THE LISTED VALUE | | | |
|---|---|---|---|
| $\sigma \leq 0.05$ | $\sigma \leq 0.062$ | $\sigma \leq 0.07$ | $\sigma \leq 0.09$ |
| 5% | 27.65% | 46.65% | 79.91% |

## FIG. 8B

| NUMBER OF DATA POINTS IN EACH COPY NUMBER GROUP: | | |
|---|---|---|
| CN=1 | 215 data points | |
| CN=2 | 799 data points | |
| CN=3 | 465 data points | |
| CN=4 | 112 data points | |
| CN=5 | 29 data points | |
| $\Delta\Delta C_T$ APPROACH WITH AUTOMATIC CALIBRATOR SAMPLE SELECTION | $\Delta\Delta C_T$ APPROACH WITH CALIBRATOR SAMPLE CHOSEN BY SCIENTIST | MAXIMUM LIKEIHOOD APPROACH BASED ON STATISTICAL MODEL |
| 76.79% | 92.90% | 83.09% |

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SCHMITTGEN et al.** *Nature Protocols,* 2008, vol. 3, 1101-1108